# EUROPÄISCHE PATENTANMELDUNG

(11) **EP 1 020 759 A1**
(43) Veröffentlichungstag der Anmeldung: **19.07.2000**
(21) Anmeldenummer: 99100344.3
(22) Anmeldetag: 14.01.1999
(51) Int. Cl.: G03B 42/04

(54) **Intraorale Halterung**

(71) Anmelder: Schäfer, Klaus, Dr. med. dent., 59067 Hamm (DE)
(72) Erfinder: Schäfer, Klaus, Dr. med. dent., 59067 Hamm (DE)
(74) Vertreter: Thul, Hermann, Dipl.-Phys.

(57) **Zusammenfassung**

Die Erfindung betrifft eine intraorale Halterung (10) mit einer Aufbißplatte (120), wobei die Halterung zu einer Lagerung eines Sensors (40) dient, der senkrecht zu der Aufbißplatte 120 gelagert wird.

Erfindungsgemäß zeichnet sich die Halterung dadurch aus, daß sie Vorsprünge (20, 30) zur Halterung des Sensors (40) aufweist.

Vorzugsweise ist die Aufbißplatte (120) so gestaltet, daß sie keilförmig ansteigt.

## Beschreibung

Die Erfindung betrifft eine intraorale Halterung mit einer Aufbißplatte, wobei die Halterung zu einer Lagerung eines Sensors dient.

Durch offenkundige Vorbenutzung sind Haltesysteme für Röntgensysteme bekannt, durch die sich mit Röntgenfilmen verschiedene Röntgenaufnahmetechniken realisieren lassen. Bei den Röntgenaufnahmetechniken handelt es sich insbesondere um die sogenannte Rechtwinkel-/Paralleltechnik, eine Kombination aus einer Rechtwinkeltechnik und einer Paralleltechnik. Die Rechtwinkel-/Paralleltechnik ermöglicht Röntgenaufnahmen mit einem Zahnfilm innerhalb der Mundhöhle.

Bei der Paralleltechnik werden Zahnachse und Filmachse parallel zueinander ausgerichtet. Trifft ein Zentralstrahl einer Röntgenquelle, insbesondere einer Röntgenröhre, auf die Mitte des Zahnes, trifft er ebenso auf die Mitte des Filmes. Die parallele Ausrichtung von Zahnachse und Filmachse gewährleistet eine Ablichtung des Zahnes auf den Film in einer im wesentlichen unveränderten Größe. Diese Aufnahmetechnik ist jedoch mit dem Nachteil einer ungenauen Justierung des Röntgentubus auf die Filmmitte sowie einer ungenauen Winkelstellung des Tubus zu dem Zahn und dem Film behaftet.

Bei der Rechtwinkeltechnik wird die Halterung so gestaltet, daß sie mit einem Tubus starr verbunden ist, so daß der Film im rechten Winkel zu dem Zentralstrahl des Röntgengerätes fixiert ist. Hierdurch trifft der Zentralstrahl stets im Winkel von 90° die Filmmitte. Diese Röntgenaufnahmetechnik hat den Vorteil, daß eine sichere Einhaltung eines rechten Winkels zwischen Zentralstrahl und Filmebene realisiert werden kann.

Bei der Rechtwinkel-/Paralleltechnik erfolgt zunächst eine Justierung des Filmes parallel zur Zahnachse und anschließend eine Ausrichtung des Tubus entlang einer Führungsstange. Hierdurch tritt der Zentralstrahl nahezu exakt im Winkel von 90° auf den Zahnfilm auf.

Der Erfindung liegt die Aufgabe zugrunde, eine Halterung für einen Sensor zu schaffen, wobei eine im wesentlichen rechtwinklige Ablichtung eines oder mehrerer Zähne auf den Sensor ermöglicht wird. Ferner soll eine möglichst genaue Justierung des Röntgentubus zu dem Sensor erzielt werden. Insbesondere soll die Halterung sich für Aufnahmen von Zahnkronen im Bereich von Ober- und Unterkiefer eignen. Vorzugsweise soll es möglich sein, den Ober- und Unterkiefer eines Patienten gleichzeitig aufzunehmen.

Erfindungsgemäß wird diese Aufgabe dadurch gelöst, daß die Halterung Vorsprünge zur Halterung des Sensors aufweist.

Durch die erfindungsgemäße Halterung werden nebeneinander liegende Zähne bei weitgehender Vermeidung von gegenseitigen Überlagerungen auf den Sensor projiziert. Hierdurch ist eine genaue Abbildung eines Berührungsbereiches von Zähnen möglich. Ferner ermöglicht die Halterung eine Projektion eines oder mehrerer Zähne auf den Sensor mit gleichbleibenden Parametern der Projektion. Die Halterung ist sowohl bei Aufnahmetechniken einsetzbar, bei denen eine genaue Größe des Zahnes oder der Zähne unmittelbar durch flächengetreue Projektion erzielt wird, als auch bei Aufnahmetechniken, bei denen eine gleichbleibende Größe erst durch eine nachfolgende Bildverarbeitung erzielt wird.

Mit Hilfe der erfindungsgemäßen Halterung ist es möglich, Röntgenstrahlen außerhalb des Sensors wirksam abzuschirmen.

Eine besonders wirksame Befestigung des Sensors läßt sich dadurch erzielen, daß wenigstens ein Vorsprung so angeordnet ist, daß er mit einer unteren Randfläche des Sensors in Kontakt gebracht werden kann und daß wenigstens ein anderer gleichlanger Vorsprung so angeordnet ist, daß er mit einer oberen Randfläche des Sensors in Kontakt gebracht werden kann.

Besonders vorteilhaft ist, daß die Halterung mit einem Visierring verbindbar ist, wobei die Verbindbarkeit insbesondere durch eine Führungsstange erzielt werden kann.

Ein wirksamer Schutz von Patienten vor Röntgenstrahlung läßt sich dadurch erzielen, daß die Halterung mit einem Befestigungsmittel für eine mögliche Befestigung einer Abschirmblende und/oder für eine mögliche Befestigung eines Visierringes verbindbar ist. Durch den Visierring kann eine im Inneren des Röntgentubus befindliche Blende zur Eingrenzung des Nutzstrahlbündels entsprechend der Sensorfläche eingestellt werden.

Vorzugsweise sind das Befestigungsmittel und die Abschirmblende so gestaltet, daß eine Öffnung der Abschirmblende im wesentlichen parallel zu dem Sensor ausrichtbar ist.

Um eine Anpassung der Halterung an anatomische Eigenschaften eines Patienten zu ermöglichen, ist es vorteilhaft, daß die Aufbißplatte im wesentlichen keilförmig ist.

Insbesondere ist es vorteilhaft, daß die Aufbißplatte derartig keilförmig geformt ist, daß sie im Bereich der Vorsprünge eine geringere Dicke aufweist als in einem Verbindungsbereich mit einer Führungsstange.

Eine unbeschädigte Zuführung mindestens eines Kabels zu dem Sensor läßt sich dadurch erzielen, daß die Aufbißplatte in wenigstens einem Bereich zwischen den Vorsprüngen und dem Kontaktbereich mit der Führungsstange eine Dicke aufweist, die wenigstens einem Durchmesser des Kabels entspricht.

Ferner ist es zweckmäßig, daß die Aufbißplatte eine zwischen den Vorsprüngen und dem Kontaktbereich mit der Führungsstange ansteigende Dicke aufweist.

Eine genaue Rechtwinkeltechnik läßt sich dadurch erzielen, daß die Aufbißplatte sich wenigstens abschnittsweise parallel zu den Vorsprüngen erstreckt.

Weitere Vorteile, Besonderheiten und zweckmäßige Weiterbildungen der Erfindung ergeben sich aus der nachfolgenden Darstellung eines bevorzugten Ausführungsbeispiels anhand der Zeichnungen.

Von den Zeichnungen zeigt:
- Fig. 1: eine Vorderansicht auf eine Halterung in einer sich parallel zu dem Sensor erstreckenden Ebene,
- Fig. 2: eine Seitenansicht der in Fig. 1 dargestellten Halterung und
- Fig. 3: eine Draufsicht auf die in den Fig. 1 und 2 dargestellte Halterung.

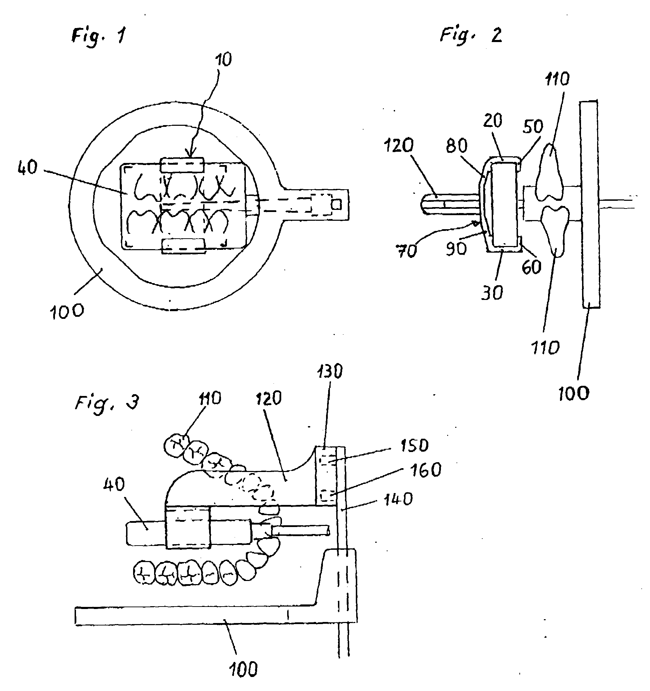

Die anhand der Fig. 1 dargestellte Halterung 10 dient insbesondere für Bißflügelaufnahmen von Zähnen. Die Halterung 10 weist Vorsprünge 20, 30 auf, die in einem Abstand voneinander angeordnet sind, der im wesentlichen so groß ist wie eine Längenausdehnung eines Sensors 40. Die Vorsprünge 20, 30 halten den Sensor 40 durch einen Formschluß. Die Vorsprünge 20, 30 sind mit seitlichen Flanken 50, 60 versehen, so daß der Formschluß verstärkt wird.

Die Vorsprünge 20, 30 sind über mindestens eine Halteplatte 70 mit einer Aufbißplatte 120 verbunden. Um ein einfaches Einführen des Sensors 40 in die Halterung 10 zu ermöglichen, ist es vorteilhaft, daß die Halteplatte 70 zwei Halteelemente 80, 90 aufweist, wobei die Halteelemente 80, 90 gespreizt sind.

Die Halterung 10 ist mit einem Visierring 100 verbindbar. Der Visierring 100 ermöglicht eine genaue Justierung eines Röntgentubus auf die Mitte des Sensors 40 und gleichzeitig auf zu untersuchende Zähne 110.

Eine Verbindung der Halterung 10 mit dem Visierring 100 erfolgt vorzugsweise über wenigstens ein Anschlußelement 130 und ein Führungsmittel 140. Das Anschlußelement 130 und das Führungsmittel 140 sind lösbar miteinander verbunden. Eine besonders einfache Verbindung zwischen der Halterung 10 und dem Visierring 100 läßt sich dadurch erzielen, daß das Anschlußelement 130 wenigstens zwei Ausnehmungen 150, 160 aufweist. In die Ausnehmungen 150, 160 können komplementär geformte Vorsprünge des Führungsmittels 140 eingebracht werden. Bei dem Führungsmittel 140 handelt es sich im einfachsten Fall um eine Führungsstange.

Eine exakte Winkellage des Sensors 40 zu den Zähnen 110 läßt sich dadurch erzielen, daß die Aufbißplatte 120 sich wenigstens abschnittsweise parallel zu den Vorsprüngen 20, 30 erstreckt.

Eine derartige Parallelität bezieht sich insbesondere darauf, daß ein dem Sensor 40 zugewandter Randbereich der Aufbißplatte 120 sich im wesentlichen parallel zu einer Hauptfläche des Sensors 40 erstreckt.

Es ist vorteilhaft, daß der Sensor 40 im wesentlichen senkrecht zu der Aufbißplatte 120 lagerbar ist.

### Bezugszeichenliste

- 10: Halterung
- 20: Vorsprung
- 30: Vorsprung
- 40: Sensor
- 50: seitliche Flanke
- 60: seitliche Flanke
- 70: Halteplatte
- 80: Halteelement
- 90: Halteelement
- 100: Visierring
- 110: Zähne
- 120: Aufbißplatte
- 130: Anschlußelement
- 140: Führungsmittel
- 150: Ausnehmung
- 160: Ausnehmung
- 170: Sensorkabel

## Patentansprüche

1. Intraorale Halterung (10) mit einer Aufbißplatte (120), wobei die Halterung zu einer Lagerung eines Sensors (40) dient, **dadurch gekennzeichnet,** daß sie Vorsprünge (20, 30) zur Halterung des Sensors (40) aufweist.

2. Halterung (10) nach Anspruch 1, **dadurch gekennzeichnet,** daß wenigstens ein Vorsprung (20) so angeordnet ist, daß er mit einer Unterseite des Sensors (40) in Kontakt gebracht werden kann und daß wenigstens ein anderer, gleichlanger Vorsprung (30) so angeordnet ist, daß er mit einer oberen Randfläche des Sensors (40) in Kontakt gebracht werden kann.

3. Halterung (10) nach einem der Ansprüche 1 oder 2, **dadurch gekennzeichnet,** daß sie über ein Führungsmittel (140) mit einem Visierring (100) verbindbar ist.

4. Halterung (10) nach einem der Ansprüche 1 bis 3, **dadurch gekennzeichnet,** daß sie mit einem Befestigungsmittel für eine Befestigung einer Abschirmblende verbindbar ist.

5. Halterung (10) nach Anspruch 4, **dadurch gekennzeichnet,** daß das Befestigungsmittel so gestaltet ist, daß eine Öffnung der Abschirmblende im wesentlichen parallel zu dem Sensor (40) ausrichtbar ist.

6. Halterung (10) nach einem der Ansprüche 1 bis 5, **dadurch gekennzeichnet,** daß die Aufbißplatte (120) im wesentlichen keilförmig ist.

7. Halterung (10) nach Anspruch 6, **dadurch gekennzeichnet,** daß die Aufbißplatte (120) derartig keilförmig geformt ist, daß sie im Bereich der Vorsprünge (20, 30) eine geringere Dicke aufweist als in einem Verbindungsbereich mit einer Führungsstange (140).

8. Halterung (10) nach einem der Ansprüche 1 bis 7, **dadurch gekennzeichnet,** daß die Aufbißplatte (120) in wenigstens einem Bereich zwischen den Vorsprüngen (20, 30) und dem Kontaktbereich mit der Führungsstange (140) eine Dicke aufweist, die wenigstens einem Durchmesser eines Sensorkabels entspricht.

9. Halterung (10) nach Anspruch 8, **dadurch gekennzeichnet,** daß die Aufbißplatte (120) eine zwischen den Vorsprüngen (20, 30) und dem Kontaktbereich mit der Führungsstange (140) ansteigende Dicke aufweist.

10. Halterung (10) nach einem der Ansprüche 1 bis 9, **dadurch gekennzeichnet,** daß die Aufbißplatte (120) so gestaltet ist, daß ihre Dicke in einem Bereich, der mit Vorderzähnen in Kontakt verbringbar ist, wenigstens einem Durchmesser des Sensorkabels entspricht.

11. Halterung (10) nach einem der Ansprüche 1 bis 10, **dadurch gekennzeichnet,** daß die Bißplatte (120) sich wenigstens abschnittsweise parallel zu den Vorsprüngen (20, 30) erstreckt.

12. Halterung (10) nach einem der Ansprüche 1 bis 11, **dadurch gekennzeichnet,** daß der Sensor (40) im wesentlichen senkrecht zu der Aufbißplatte (120) lagerbar ist.
